# EUROPEAN PATENT APPLICATION

(11) **EP 4 838 467 A1**
(43) Date of publication of application: **11.11.2026**
(21) Application number: 25891104.9
(22) Date of filing: 29.10.2025
(51) Int. Cl.: G06T 7/62

(54) **LIMB VOLUME MEASUREMENT METHOD, APPARATUS AND DEVICE, AND STORAGE MEDIUM**

(30) Priority: 14.11.2024 CN 202411625556
(71) Applicant: Sun Yat-Sen Memorial Hospital , Sun Yat-Sen University, Guangzhou, Guangdong 510000 (CN); Guangzhou Breast-Mimics Medicine Technology Limited Company, Guangzhou, Guangdong 510700 (CN); Zenith Institute of Medical Sciences, Guangzhou, Guangzhou, Guangdong 510700 (CN)
(72) Inventor: WU, Wei, Guangzhou, Guangdong 510000 (CN); DENG, Heran, Guangzhou, Guangdong 510000 (CN); YAN, Jingwen, Guangzhou, Guangdong 510000 (CN); WU, Jiannan, Guangzhou, Guangdong 510000 (CN); YAO, Pengju, Guangzhou, Guangdong 510700 (CN)
(74) Representative: Manitz Finsterwald Patent- und Rechtsanwaltspartnerschaft mbB
(86) International application number: PCT/CN2025/130998
(87) International publication number: WO 2026/103514

(57) **Abstract**

Disclosed in the embodiments of the present application are a limb volume measurement method, apparatus and device, and a storage medium. The method is used for a medical measurement device provided with at least four cameras, the cameras being arranged into a circle at preset intervals, and camera lenses facing the central area of the circle. The method comprises: when it is detected that a limb to be measured passes through the inside of the circle enclosed by the cameras, controlling the cameras to perform mobile scanning on said limb, and performing deduplication and fusion on point cloud data obtained by scanning, so as to obtain fused point cloud data; performing three-dimensional modelling on the basis of the fused point cloud data to obtain a target three-dimensional limb model; slicing the target three-dimensional limb model and performing center-of-gravity analysis on same to determine a joint center-of-gravity point; reslicing on the basis of the joint center-of-gravity point to obtain target slice data; and integrating volume information of each piece of target slice data to obtain overall volume information. The present application can solve the problems of low work efficiency and large errors in the existing limb volume measurement and can automatically scan and identify limb volumes, thereby improving the work efficiency and accuracy of measuring limb volumes.

## Description

### TECHNICAL FIELD

Embodiments of the present application relate to the technical field of limb volume detection, and in particular, to a method and an apparatus for limb volume detection, a device, and a storage medium.

### BACKGROUND

Edema refers to the excessive accumulation of body fluid in the interstitial spaces outside the blood vessels, and is a common pathological process. Edema often signals certain internal diseases, such as hypertension, diabetes, and congestive heart disease, and therefore the detection of edema is of significant clinical importance. At present, the volume information of a limb can be measured to determine whether edema occurs by comparing changes in the volume information.

Existing methods of measuring edema mainly rely on medical staff manually measuring with a soft tape. Manual measurement not only has low working efficiency, but also causes a relatively large error in determining whether edema occurs for the reason that the tightness of the soft tape differs in each measurement.

### SUMMARY

Embodiments of the present application provide a method and an apparatus for limb volume detection, a device, and a storage medium, which can solve the problems of low working efficiency and large error of the existing limb volume detection, can automatically scan and identify limb volume, improve the working efficiency of limb volume detection, and improve the accuracy of limb volume measurement.

In a first aspect, an embodiment of the present application provides a method for limb volume detection, for use in a medical detection device. The medical detection device is provided with at least four cameras arranged circumferentially at preset intervals around a ring, lenses of the cameras face toward a central region of the ring, and the ring is configured for a limb to be detected to pass through. The method for limb volume detection specifically comprises:
upon a detection that the limb to be detected has passed through the interior of the ring surrounded by the cameras, controlling all cameras to simultaneously move along the limb to be detected and perform scanning to obtain point cloud data acquired by scanning with the plurality of cameras;
performing deduplication and fusion processing on the point cloud data based on a preset spatial calibration relationship to obtain fused point cloud data of the cameras, and the preset spatial calibration relationship being a relative positional relationship between every two cameras;
performing three-dimensional modeling processing based on the fused point cloud data to obtain a target three-dimensional limb model;
performing slicing processing and centroid analysis processing based on the target three-dimensional limb model to determine a joint centroid point;
performing re-slicing processing on the target three-dimensional limb model based on the joint centroid point to obtain target slice data;
performing parameter calculation processing based on the target slice data to obtain volume information of each piece of target slice data, and the volume information including a slice thickness and a cross-sectional area; and
performing integration processing on volume parameters of each piece of target slice data to obtain overall volume information of the limb to be detected.

In an embodiment, performing the deduplication and fusion processing on the point cloud data based on the preset spatial calibration relationship to obtain the fused point cloud data of the cameras, and the preset spatial calibration relationship being the relative positional relationship between every two cameras, comprises:
determining an overlapping region in regions corresponding to the point cloud data based on the preset spatial calibration relationship; and
performing the deduplication processing on the point cloud data of the overlapping region to obtain the fused point cloud data, wherein the fused point cloud data comprises point cloud data of a non-overlapping region, and the processed point cloud data of the overlapping region obtained after the deduplication processing.

In an embodiment, performing the three-dimensional modeling processing based on the fused point cloud data to obtain the target three-dimensional limb model comprises:
creating an initial voxel grid based on a resolution of the fused point cloud data;
performing voxelization processing on the fused point cloud data based on the initial voxel grid to obtain first voxel data, and the first voxel data including vertices, voxel indices, and a voxel value corresponding to each voxel; and
mapping the first voxel data into a point cloud mesh, and performing color rendering processing based on a mapping result and a projection superposition result to generate the target three-dimensional limb model.

In an embodiment, performing the slicing processing and the centroid analysis processing based on the target three-dimensional limb model to determine the joint centroid point comprises:
performing the slicing processing on the target three-dimensional limb model along a preset direction at preset distances to obtain reference slice data;
calculating and determining a centroid point of each piece of the reference slice data;
connecting a centroid point of a first piece of the reference slice data with a centroid point of a last piece of the reference slice data to obtain a first reference line; and
determining, among centroid points of all the reference slice data, a centroid point farthest from the first reference line as the joint centroid point.

In an embodiment, performing the re-slicing processing on the target three-dimensional limb model based on the joint centroid point to obtain the target slice data comprises:
connecting the centroid point of the first piece of the reference slice data with the joint centroid point to obtain a second reference line;
performing the re-slicing processing on a first portion of the target three-dimensional limb model along the second reference line at preset intervals to obtain first target slice data, wherein the first portion is a portion between starting point cloud of the first piece of the reference slice data and ending point cloud in the reference slice data corresponding to the joint centroid point;
connecting the joint centroid point with the centroid point of the last piece of the reference slice data to obtain a third reference line;
performing the re-slicing processing on a second portion of the target three-dimensional limb model along the third reference line at preset intervals to obtain second target slice data, wherein the second portion is a portion between starting point cloud in the reference slice data corresponding to the joint centroid point and ending point cloud of the last piece of the reference slice data; and
performing fusion and stitching processing on the first target slice data and the second target slice data using the joint centroid point as a stitching point to obtain overall target slice data of the target three-dimensional limb model.

In an embodiment, before performing the slicing processing and the centroid analysis processing based on the target three-dimensional limb model to determine the joint centroid point, the method comprises:
performing two scans and two three-dimensional modeling processing operations on the limb to be detected to obtain a corresponding first three-dimensional limb model and a second three-dimensional limb model, respectively;
performing the slicing processing on the first three-dimensional limb model and the second three-dimensional limb model respectively along a preset direction at preset intervals, to obtain first slice data corresponding to the first three-dimensional limb model and second slice data corresponding to the second three-dimensional limb model;
performing centroid point calculation processing on the first slice data to obtain first centroid point data, wherein the first centroid point data comprises centroid points of all the first slice data;
performing centroid point calculation processing on the second slice data to obtain second centroid point data, wherein the second centroid point data comprises centroid points of all the second slice data;
mapping the first centroid point data and the second centroid point data into a same plane for curve plotting to obtain a first curve and a second curve, respectively;
performing distance calculation processing based on the first curve and the second curve to obtain a Euclidean distance between the first curve and the second curve;
determining the first three-dimensional limb model as the target three-dimensional limb model when the Euclidean distance does not exceed a preset value; and
performing re-scanning and three-dimensional re-modeling processing on the limb to be detected when the Euclidean distance exceeds the preset value, until the Euclidean distance does not exceed the preset value, and determining a corresponding first three-dimensional limb model as the target three-dimensional limb model.

In an embodiment, the method for limb volume detection further comprises:
upon a detection that the limb to be detected has passed through the interior of the ring surrounded by the cameras, controlling all the cameras to simultaneously move along the calibration object and perform scanning to obtain reference point cloud data;
comparing the reference point cloud data with standard point cloud data to determine a first positional relationship of each camera relative to the calibration object, wherein the standard point cloud data is obtained by performing point cloud calibration and correction on the calibration object;
performing grouped calibration on the spatial positional relationships of the cameras based on the first positional relationship to determine a second positional relationship between the cameras within a corresponding group;
performing inter-group registration processing on the spatial positional relationships of the cameras based on the first positional relationship and the second positional relationship to obtain a third relative positional relationship between every two groups; and
performing pairwise calibration processing on the spatial positional relationships of the cameras based on the first positional relationships, the second positional relationships, and the third relative positional relationship to obtain the preset spatial calibration relationships between all the cameras.

In a second aspect, an embodiment of the present application provides an apparatus for limb volume detection, for use in a medical detection device. The medical detection device is provided with at least four cameras arranged circumferentially at preset intervals around a ring, lenses of the cameras face toward a central region of the ring, and the ring is configured for a limb to be detected to pass through. The apparatus comprises:
a scanning module, configured to, upon a detection that the limb to be detected has passed through the interior of the ring surrounded by the cameras, control all cameras to simultaneously move along the limb to be detected and perform scanning to obtain point cloud data acquired by scanning with the plurality of cameras; a point cloud fusion module, configured to perform deduplication and fusion processing on the point cloud data based on a preset spatial calibration relationship to obtain fused point cloud data of the cameras, wherein the preset spatial calibration relationship is a relative positional relationship between every two cameras;
a modeling module, configured to perform three-dimensional modeling processing based on the fused point cloud data to obtain a target three-dimensional limb model;
a joint determination module, configured to perform slicing processing and centroid analysis processing based on the target three-dimensional limb model to determine a joint centroid point;
a re-slicing module, configured to perform re-slicing processing on the target three-dimensional limb model based on the joint centroid point to obtain target slice data;
a first calculation module, configured to perform parameter calculation processing based on the target slice data to obtain volume information of each piece of target slice data, wherein the volume information comprises a slice thickness and a cross-sectional area; and
a second calculation module, configured to perform integration processing on volume parameters of each piece of target slice data to obtain overall volume information of the limb to be detected.

In a third aspect, an embodiment of the present application provides a device for limb volume detection, comprising: a memory and one or more processors.

The memory is configured to store one or more programs.

The one or more programs, when executed by the one or more processors, cause the one or more processors to perform the method for limb volume detection of the first aspect.

In a fourth aspect, an embodiment of the present application provides a storage medium having computer-executable instructions stored thereon. The computer-executable instructions, when executed by a processor, are used to perform the method for limb volume detection of the first aspect.

Embodiments of the present application are used for a medical detection device provided with at least four cameras, the cameras are arranged at preset intervals to form the ring, the lenses of the cameras face toward the central region of the ring, and the ring is configured for the limb to be detected to pass through. Upon the detection that the limb to be detected has passed through the interior of the ring surrounded by the cameras, all the cameras are controlled to simultaneously move along the limb to be detected and perform scanning. The deduplication and fusion processing as well as the three-dimensional modeling processing are performed based on the point cloud data obtained by scanning to obtain the target three-dimensional limb model. The slicing processing and the centroid analysis processing are performed based on the target three-dimensional limb model to determine the joint centroid point. The re-slicing processing is performed on the target three-dimensional limb model based on the joint centroid point to obtain the target slice data. The parameter calculation and the data integration are performed based on the target slice data to obtain the overall volume information of the limb to be detected. By adopting the above technical solutions, the overall volume information of the limb to be detected can be obtained simply by scanning with the medical detection device, thereby avoiding the technical problems of low working efficiency and large error caused by manual measurement. Moreover, the overall volume information of the limb to be detected can be obtained based on camera scanning, thereby improving the working efficiency of the limb volume detection. Moreover, the preliminary slicing is first performed to determine the joint centroid point, and the re-slicing is performed based on the joint centroid point, with slicing being carried out separately using the joint centroid point as a boundary, thereby improving the accuracy of slices and improving the accuracy of the volume calculation.

### BRIEF DESCRIPTION OF THE DRAWINGS

FIG. 1 is a schematic view showing a distribution of a single group of cameras provided by an embodiment of the present application;
FIG. 2 is a schematic view showing a positional relationship between cameras and a limb provided by an embodiment of the present application;
FIG. 3 is a flowchart of a method for limb volume detection provided by an embodiment of the present application;
FIG. 4 is a schematic view showing a positional relationship between cameras and a calibration object provided by an embodiment of the present application;
FIG. 5 is a first schematic view showing slices of a limb model provided by an embodiment of the present application;
FIG. 6 is a second schematic view showing slices of a limb model provided by an embodiment of the present application;
FIG. 7 is a schematic view showing a structure of an apparatus for limb volume detection provided by an embodiment of the present application;
FIG. 8 is a schematic view showing a structure of a device for limb volume detection provided by an embodiment of the present application.

### DETAILED DESCRIPTION OF THE EMBODIMENTS

In order to make the objectives, technical solutions, and advantages of the present application clearer, the specific embodiments of the present application are further described in detail below with reference to the accompanying drawings. It should be understood that the specific embodiments described herein are merely used to explain the present application and are not intended to limit the present application. In addition, it should also be noted that, for ease of description, the accompanying drawings show only some, rather than all, of the content related to the present application. Before discussing the exemplary embodiments in more detail, it should be mentioned that some exemplary embodiments are described as processes or methods depicted as flowcharts. Although a flowchart describes various operations (or steps) as sequential processing, many of the operations therein may be performed in parallel, concurrently, or simultaneously. In addition, the order of the various operations may be rearranged. The processing may be terminated when its operations are completed, but may also have additional steps not included in the accompanying drawings. The processing may correspond to a method, a function, a procedure, a subroutine, a subprogram, and so on.

Edema refers to the excessive accumulation of body fluid in the interstitial spaces outside the blood vessels, and is a common pathological process. Edema often signals certain internal diseases, such as hypertension, diabetes, and congestive heart disease, and therefore the detection of edema is of significant clinical importance. At present, the volume information of a limb can be measured to determine whether edema occurs by comparing changes in the volume information. Existing methods of measuring edema mainly rely on medical staff manually measuring with a soft tape. Manual measurement not only has low working efficiency, but also causes a relatively large error in determining whether edema occurs for the reason that the tightness of the soft tape differs in each measurement.

At present, three-dimensional scanners can also be used for detecting a limb volume. However, in existing methods, slicing is performed directly along the axial direction of the three-dimensional scanner. Because a limb of a test subject is curved, taking an arm as an example, the forearm and the upper arm are not on the same straight line during detection, or the placement direction of the arm is not parallel to the axial direction of the three-dimensional scanner. In this case, measuring the slice parameters through slicing along the axial direction of the three-dimensional scanner will result in a certain error, thus causing poor accuracy of the detection results.

Based on this, embodiments of the present application provide methods for limb volume detection to solve the technical problems of low working efficiency and large error in the existing limb volume detection process. With the method for limb volume detection provided by the present application, the overall volume information of the limb to be detected can be obtained simply by scanning with a medical detection device, thereby avoiding the technical problems of low working efficiency and large error caused by manual measurement. The overall volume information of the limb to be detected can be obtained based on camera scanning, which, compared with existing manually measuring methods, improves the working efficiency of limb volume detection. Moreover, preliminary slicing is first performed to determine the joint centroid point, and re-slicing is performed based on the joint centroid point, with slicing being carried out separately by using the joint centroid point as a boundary, which, compared with the existing methods of slicing directly along the axial direction of the scanner, improves slicing accuracy, thereby improving the accuracy of volume calculation.

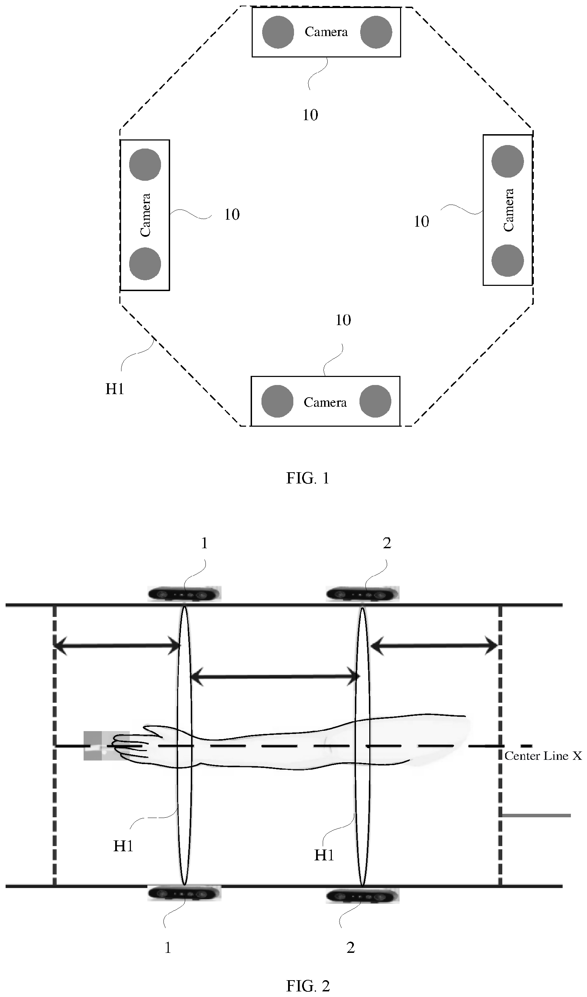

The methods for limb volume detection provided by embodiments of the present application are used for a medical detection device. The medical detection device is provided with at least four cameras, and the cameras are arranged circumferentially at preset intervals around a ring, and the cameras on each ring constitute a camera group. Each camera is a binocular camera. FIG. 1 is a schematic view showing a distribution of a single group of cameras provided by an embodiment of the present application. Referring to FIG. 1, the medical detection device is provided with four cameras 10, and every four cameras constitute a camera group. Taking one camera group as an example for illustration, the four cameras 10 within the group are arranged circumferentially at preset intervals around a ring, the lenses of the four cameras 10 face toward a central region of the ring H1, and the ring H1 is configured for a limb to be detected to pass through. The medical detection device provided by the embodiment is provided with at least one camera group, and each camera group is provided with at least four cameras 10. FIG. 2 is a schematic view showing a positional relationship between cameras and a limb provided by an embodiment of the present application. Referring to FIG. 2, taking the arrangement of two camera groups in the medical detection device as an example for illustration, the two camera groups are a camera group 1 and a camera group 2, respectively. In camera group 1 and camera group 2, the four cameras 10 within each group are, as shown in FIG. 1, arranged circumferentially at preset intervals around a ring, the lenses of the four cameras 10 face toward the central region of the ring H1, and the ring H1 is configured for a limb to be detected to pass through. It should be noted that, FIG. 2 is illustrated by taking an arm as an example of the limb to be detected. However, in actual detection, the limb to be detected may also be a limb other than an arm, such as a leg. As shown in FIG. 2, the camera group 1 and the camera group 2 are arranged at a preset distance. The limb to be detected passes through the interior of the ring H1 formed by the cameras 10 of each camera group, and is located inside the ring H1 and approximately at the position of a center line X thereof. Upon the detection that the limb to be detected has passed through the interior of the ring H1 formed by the cameras 10, all the cameras 10 are controlled to simultaneously move along the limb to be detected and perform scanning.

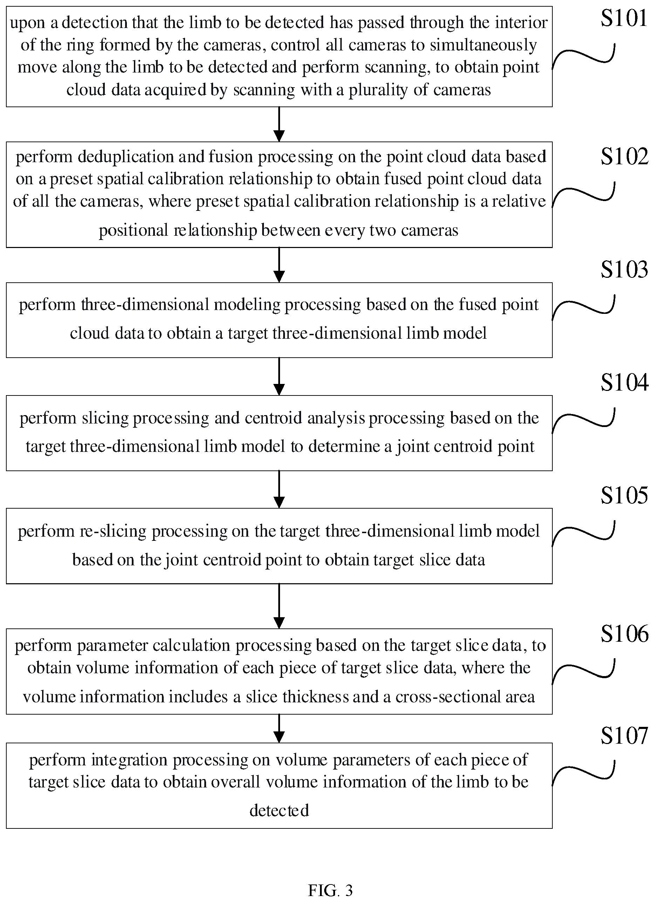

FIG. 3 shows a flowchart of a method for limb volume detection provided by an embodiment of the present application. The method for limb volume detection provided in this embodiment may be performed by a device for limb volume detection, which may be implemented by means of software and/or hardware. The device for limb volume detection may be constituted by two or more physical entities, or may be constituted by a single physical entity. Generally speaking, the device for limb volume detection may be a medical detection device, such as a three-dimensional scanner.

The following description is provided by taking the medical detection device as a subject performing the method for limb volume detection as an example. Referring to FIG. 3, the method for limb volume detection is used for a medical detection device, the medical detection device is provided with at least four cameras, the cameras are arranged circumferentially at preset intervals around the ring, the lenses of the cameras face toward the central region of the ring, and the ring is configured for a limb to be detected to pass through. The method for limb volume detection specifically includes steps S101 to S107.

In step S101, upon a detection that the limb to be detected has passed through the interior of the ring surrounded by the cameras, control all cameras to simultaneously move along the limb to be detected and perform scanning, to obtain point cloud data acquired by scanning with the plurality of cameras.

The medical detection device provided by an embodiment is as shown in FIG. 1 and FIG. 2. The medical detection device includes at least one camera group, the four cameras 10 within each camera group are arranged circumferentially at preset intervals around a ring, the lenses of the four cameras 10 face toward the central region of the ring H1, and the ring H1 is configured for the limb to be detected to pass through. When it is detected that the limb to be detected passes through the interior of the ring H1 surrounded by the cameras 10, for example as shown in FIG. 2, all the cameras 10 are controlled to move along the limb to be detected and perform scanning, so as to obtain the point cloud data acquired by scanning with the plurality of cameras 10. It should be noted that, due to overlapping fields of view among the cameras 10, the point cloud data acquired by scanning with the plurality of cameras 10 also contains corresponding overlapping point cloud data.

In the foregoing, upon the detection that the limb to be detected is placed inside the ring surrounded by the cameras, all the cameras are controlled to simultaneously move along the limb to be detected and scan, so as to obtain the scanned point cloud data. Subsequently, three-dimensional modeling can be performed based on the point cloud data, and limb volume detection can be performed based on the three-dimensional limb model obtained by modeling. Compared with the existing method of measuring with a soft tape, the method of this embodiment, which is based on the three-dimensional limb model obtained based on camera scanning, achieves higher accuracy, and automatically realizes scanning and volume detection, thereby improving the degree of automation of the limb volume detection, and further improving the working efficiency of the limb volume detection.

In step S102, perform deduplication and fusion processing on the point cloud data based on a preset spatial calibration relationship to obtain fused point cloud data of all the cameras. The preset spatial calibration relationship is a relative positional relationship between every two cameras.

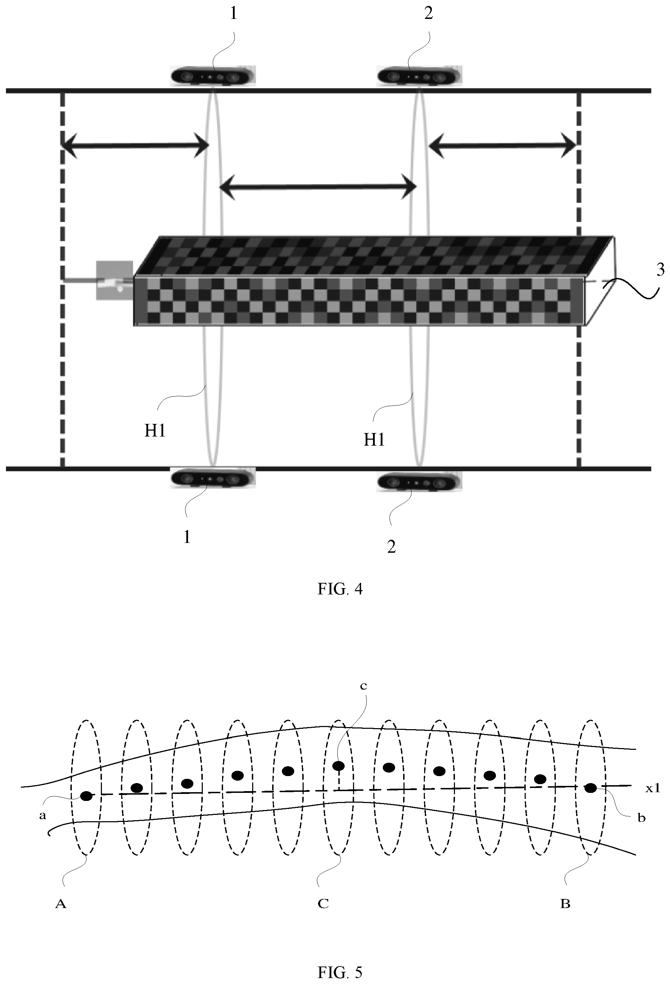

Before formal detection, the spatial positions of the cameras need to be calibrated to obtain the preset spatial calibration relationship of all cameras, i.e., the relative positional relationship between the cameras. During subsequent formal detection, deduplication processing and fusion processing are performed on the point clouds based on the preset spatial calibration relationship between all the cameras. An embodiment provides a calibration method for a preset spatial calibration relationship, which specifically includes: upon a detection that the limb to be detected has passed through the interior of the ring surrounded by the cameras, controlling all the cameras to simultaneously move along the calibration object and perform scanning, so as to obtain reference point cloud data. In three-dimensional modeling, a calibration object (also referred to as a calibration member) is a specific object or device used for calibrating, verifying, or standardizing a measurement system, a camera system, a scanning device, or other three-dimensional data acquisition device. The design and use of the calibration member are intended to ensure that these devices can accurately and reliably acquire and reconstruct the shape, dimensions, and positional information of objects in three-dimensional space. FIG. 4 is a schematic view showing a positional relationship between cameras and a calibration object provided by an embodiment of the present application. Referring to FIG. 4, upon the detection that a calibration object 3 has passed through the interior of the ring H1 formed by the cameras 10, all the cameras 10 are controlled to simultaneously move along the calibration object 3 and perform scanning, so as to obtain the reference point cloud data. The reference point cloud data is compared with standard point cloud data to determine a first positional relationship of each camera 10 relative to the calibration object 3. The standard point cloud data is obtained by performing point cloud calibration and correction on the calibration object 3. Grouped calibration is performed on the spatial positional relationships of the cameras 10 based on the first positional relationships thereof, so as to determine a second positional relationship between the cameras 10 within the corresponding group. Inter-group registration processing is performed on the spatial positional relationships of the cameras 10 based on the first positional relationships and the second positional relationships, so as to obtain a third relative positional relationship between every two groups. For example, as shown in FIG. 2, the relationship between a camera group 1 and a camera group 2 is registered, and the third positional relationship between the camera group 1 and the camera group 2 is calibrated. Pairwise calibration processing is performed on the spatial positional relationships of the cameras 10 based on the first positional relationships, the second positional relationships, and the third relative positional relationship, so as to obtain the preset spatial calibration relationships between the cameras 10. The result finally obtained by calibration (i.e., the preset spatial calibration relationships) are stored in a system file, and during subsequent volume detection of the limb to be detected, the pre-stored preset spatial calibration relationships can be directly invoked to perform deduplication and fusion processing on the point cloud data, thereby improving the overall working efficiency of the limb volume detection.

An overlapping region in the regions corresponding to the point cloud data is determined based on the preset spatial calibration relationships, and the deduplication processing is performed on the point cloud data of the overlapping region to obtain the fused point cloud data. The fused point cloud data includes point cloud data of a non-overlapping region, and the processed point cloud data of the overlapping region obtained after the deduplication processing. The fused point cloud data may be understood as the fused point cloud data of the limb to be detected, and subsequently, the three-dimensional modeling is performed based on the fused point cloud data to obtain the three-dimensional limb model of the limb to be detected.

After scanning the limb to be detected to obtain the point cloud data, the deduplication processing and the fusion processing are performed on the point cloud data based on the preset spatial calibration relationship, so as to obtain the fused point cloud data of all the cameras, i.e., the fused point cloud data of the limb to be detected, thereby realizing deduplication and fusion, improving the accuracy of the point cloud data, improving the accuracy of the three-dimensional limb model obtained by the subsequent three-dimensional modeling, and further improving the accuracy of the limb volume detection.

In step S103, perform three-dimensional modeling processing based on the fused point cloud data to obtain a target three-dimensional limb model.

The three-dimensional modeling processing is performed based on the fused point cloud data obtained as described above, so as to obtain the target three-dimensional limb model. Exemplarily, an initial voxel grid may be created based on a resolution of the fused point cloud data. Voxelization processing is performed on the fused point cloud data based on the initial voxel grid to obtain first voxel data. The first voxel data includes vertices, voxel indices, and a voxel value corresponding to each voxel. The first voxel data is mapped into a point cloud mesh, and color rendering processing is performed based on a mapping result and a projection superposition result to generate the target three-dimensional limb model.

In the foregoing, by detecting that the limb to be detected is placed inside the ring surrounded by the cameras, all the cameras are controlled to scan to obtain the point cloud data, and the point cloud data is subjected to the deduplication processing and the fusion processing to obtain the fused point cloud data. The three-dimensional modeling processing is performed based on the fused point cloud data to obtain the target three-dimensional limb model, i.e., the three-dimensional limb model corresponding to the limb to be detected. Subsequently, calculation processing can be directly performed on the target three-dimensional limb model to obtain the corresponding volume information. Compared with the existing method of measuring with a soft tape, the method of this embodiment, which is based on the three-dimensional limb model obtained by camera scanning, achieves higher accuracy, and automatically realizes scanning and volume detection, thereby improving the degree of automation of the limb volume detection, and further improving the working efficiency of the limb volume detection.

Before performing the volume detection (calculation) on the target three-dimensional limb model, it is necessary to confirm whether the limb has undergone severe shaking. If severe shaking occurs, the three-dimensional limb model obtained based on the point cloud data may have a relatively large error, which would affect the accuracy of the limb volume obtained by subsequent calculation. Therefore, it is necessary to determine that a shaking range of the limb to be detected is within a threshold range (i.e., within a range in which the error caused by shaking can be ignored) before performing limb volume detection based on the target three-dimensional limb. If it is determined that the shaking range of the limb to be detected exceeds the threshold range, this indicates that the limb to be detected shook severely during the scanning process and needs to be re-measured.

An embodiment provides a limb shaking detection method, which specifically includes: performing two scans and two three-dimensional modeling processing operations on the limb to be detected, so as to obtain a first three-dimensional limb model and a second three-dimensional limb model, respectively. The specific scanning and modeling processes of the first three-dimensional limb model and the second three-dimensional limb model are the same as those of S101 to S103 described above and will not be repeatedly described here. By means of the first three-dimensional limb model and the second three-dimensional limb model obtained based on scanning the same limb to be detected twice, the first three-dimensional limb model and the second three-dimensional limb model are compared to confirm whether the limb to be detected shook during measurement. By performing slicing processing on the first three-dimensional limb model and the second three-dimensional limb model respectively along a preset direction at preset intervals (for example, 1 cm), first slice data corresponding to the first three-dimensional limb model and second slice data corresponding to the second three-dimensional limb model are obtained. The preset direction may be the direction of the center line of the ring surrounded by the cameras (which may also be referred to as the axial direction of the medical detection device), the placement direction of the limb to be detected, or the placement direction of a preset calibration object, etc. Centroid point calculation processing is performed on the first slice data to obtain first centroid point data, and the first centroid point data includes the centroid points of all the first slice data. Centroid point calculation processing is performed on the second slice data to obtain second centroid point data, and the second centroid point data includes the centroid points of all the second slice data. The first centroid point data and the second centroid point data are mapped into a same plane for curve plotting to obtain a first curve and a second curve respectively. The first curve corresponds to the variation of the slice centroid points of the first three-dimensional limb model, and the second curve corresponds to the variation of the slice centroid points of the second three-dimensional limb model. Distance calculation processing is performed based on the first curve and the second curve to obtain a Euclidean distance between the first curve and the second curve. The degree of coincidence of the two curves is determined based on the Euclidean distance. When the Euclidean distance does not exceed a preset value, it indicates that the shaking of the limb to be detected falls within a normal range, and the first three-dimensional limb model is determined as the target three-dimensional limb model. When the Euclidean distance exceeds the preset value, it indicates that severe shaking occurred during the scanning of the limb to be detected and that the corresponding three-dimensional limb model obtained by scanning and modeling is not sufficiently accurate. Therefore, the limb to be detected needs to be re-scanned, and the three-dimensional re-modeling processing needs to be performed, until the Euclidean distance does not exceed the preset value, and the corresponding first three-dimensional limb model is determined as the target three-dimensional limb model.

In the foregoing, by means of the limb shaking detection method, the three-dimensional limb model corresponding to a shaking within the normal range is determined as the target three-dimensional limb model, thereby avoiding analyzing and calculating based on a three-dimensional limb model obtained when the limb to be detected shook severely, which would result in inaccurate final volume information. In this embodiment, by determining the three-dimensional limb model corresponding to the shaking within the normal range as the target three-dimensional limb model, the accuracy of the target three-dimensional limb model can be improved, thereby improving the accuracy of the volume information obtained by volume detection performed subsequently based on the target three-dimensional limb model.

In step S104, perform slicing processing and centroid analysis processing based on the target three-dimensional limb model to determine a joint centroid point.

After determining the target three-dimensional limb model corresponding to the limb to be detected, the target three-dimensional limb model may be decomposed into slices by means of slicing, volume detection is performed for each slice, and the volume parameters corresponding to all the slices are integrated to obtain the complete volume information corresponding to the limb to be detected. However, during the scanning process, the limb to be detected may not be placed completely straight and may form a certain angle. Taking the limb to be detected being an arm as an example for illustration, because the arm has a joint position, it is not completely straight during scanning and is curved to a certain extent at the joint position. Therefore, in the existing method of obtaining the limb volume by performing a single slicing calculation on the three-dimensional limb model directly along the placement direction of the arm, the curving of the arm causes a corresponding error in the slice data. Accordingly, an embodiment provides a two-stage slicing method: the first slicing is performed along a preset direction to obtain slice data, and analysis processing is performed based on the slice data to determine the joint position, and then re-slicing is performed based on the joint position, with slicing carried out in corresponding directions using the joint position as a boundary, so as to reduce the influence of the curving at the joint position on the accuracy of the slice data, thereby improving the accuracy of the final slice data.

An embodiment provides an implementation for determining the joint position in the target three-dimensional limb model, which specifically includes: performing the slicing processing on the target three-dimensional limb model along a preset direction at preset distances to obtain reference slice data; calculating and determining a centroid point of each piece of the reference slice data; connecting a centroid point of a first piece of the reference slice data with a centroid point of a last piece of the reference slice data to obtain a first reference line; and determining, among the centroid points of all the reference slice data, the centroid point farthest from the first reference line as the joint centroid point. Exemplarily, FIG. 5 is a first schematic view showing slices of a limb model provided by an embodiment of the present application. Referring to FIG. 5, the target three-dimensional limb model is sliced along the preset direction (assumed to be the X1 axis direction (not shown in the figure) corresponding to the first reference line x1 in the figure) at preset distances (assumed to be 1 cm) to obtain the reference slice data. It is assumed that the first reference slice obtained is slice *A* and the last reference slice is slice *B*. The centroid point of each piece of reference slice data is calculated and determined. It is assumed that the centroid point *a* of slice *A* and the centroid point *b* of slice *B* are obtained by calculation, and the centroid point *a* and the centroid point *b* are connected to obtain the first reference line x1. Among the centroid points of all the reference slice data, the centroid point farthest from the first reference line x1 is determined as the joint centroid point. Assuming that the centroid point farthest from the first reference line x1 is point *c*, the joint centroid point is determined to be the point *c*.

In the foregoing, the joint centroid point is determined by preforming slicing processing and centroid analysis processing based on the target three-dimensional limb model, and subsequently a second slicing is performed based on the joint centroid point, so as to reduce the influence of the curving at the joint position on the accuracy of the slice data, thereby improving the accuracy of the slice data and improving the accuracy of the volume information calculated based on the slice data.

In step S105, perform re-slicing processing on the target three-dimensional limb model based on the joint centroid point to obtain target slice data.

After determining the joint centroid point as described above, the target three-dimensional limb model is divided into two portions by using the joint centroid point as a boundary: the portion between starting point cloud of the first piece of reference slice data and ending point cloud in the reference slice data corresponding to the joint centroid point is defined as a first portion, for example the forearm of the arm; the portion between starting point cloud in the reference slice data corresponding to the joint centroid point and ending point cloud of the last piece of reference slice data is defined as a second portion, for example the upper arm of the arm. The first portion and the second portion may be re-sliced separately, such that the first portion and the second portion are sliced along their respective axial directions, thereby avoiding the situation where, when the whole target three-dimensional limb is sliced along a single direction, the curving at the joint position affects the accuracy of the slice data. Specifically, the centroid point of the first piece of reference slice data and the joint centroid point are connected to obtain a second reference line. The first portion of the target three-dimensional limb model is re-sliced along the second reference line at preset intervals to obtain first target slice data. The joint centroid point and the center point of the last piece of reference slice data are connected to obtain a third reference line. The second portion of the target three-dimensional limb model is re-sliced along the third reference line at preset intervals to obtain second target slice data. The first target slice data and the second target slice data are subjected to fusion and stitching processing using the joint centroid point as a stitching point to obtain the overall target slice data of the target three-dimensional limb model.

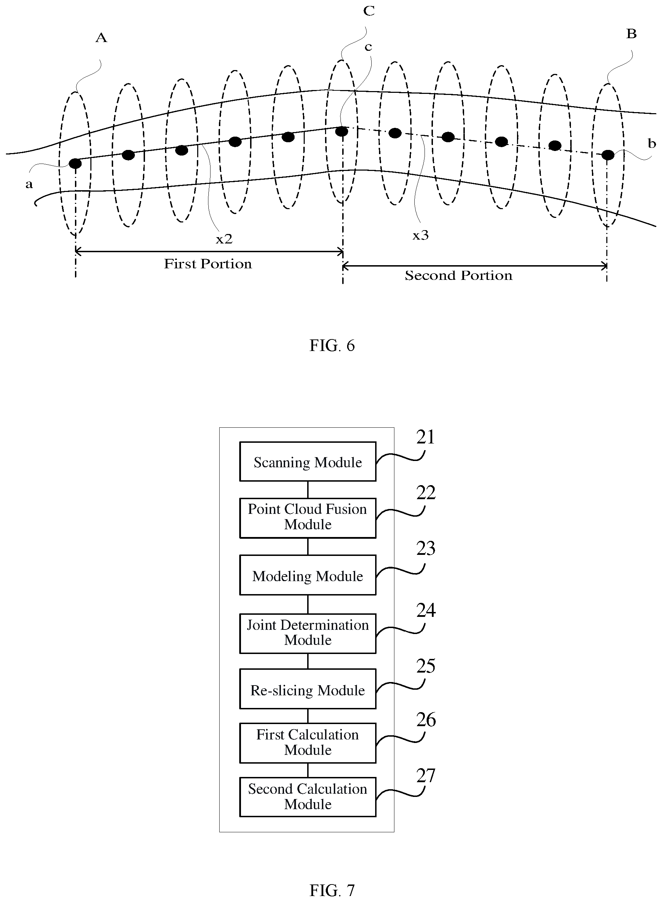

Exemplarily, FIG. 6 is a second schematic view showing slices of a limb model provided by an embodiment of the present application. Referring to FIG. 6, the joint centroid point is determined to be the point *c*, and the reference slice corresponding to the joint centroid point is slice *C*. The portion from the slice *A* to the slice *C* is defined as the first portion, and the portion from the slice *C* to the slice *B* is defined as the second portion. The centroid point *a* of slice *A* and the joint centroid point *c* are connected to obtain the second reference line X2. The first portion of the target three-dimensional limb model (the portion from the slice *A* to the slice *C*) is re-sliced along the second reference line X2 at the preset intervals (for example, 1 cm) to obtain the first target slice data. The joint centroid point *c* and the centroid point *b* of the reference slice *B* are connected to obtain the third reference line X3. The second portion of the target three-dimensional limb model (the portion from the slice *C* to the slice *B*) is re-sliced along the third reference line X3 at the preset intervals (for example, 1 cm) to obtain the second target slice data. The first target slice data and the second target slice data are subjected to the fusion and stitching processing using the joint centroid point as the stitching point, so as to obtain the overall target slice data of the target three-dimensional limb model.

In step S106, perform parameter calculation processing based on the target slice data, to obtain volume information of each piece of target slice data. The volume information includes a slice thickness and a cross-sectional area.

After obtaining the target slice data by performing the re-slicing processing based on the joint centroid point as described above, the parameter calculation processing is performed based on the target slice data to obtain the slice thickness and the cross-sectional area corresponding to each piece of target slice data, i.e., to obtain the volume information of each piece of target slice data. Because the limb to be detected is not a regular cylindrical object, the embodiment, by means of slicing, the target three-dimensional limb model corresponding to the limb to be detected is sliced into slices that are approximately cylindrical, and the volume information of each slice is calculated, which makes the calculation more convenient and accurate and thereby improves the overall working efficiency and accuracy of the volume detection.

In step S107, perform integration processing on volume parameters of each piece of target slice data to obtain overall volume information of the limb to be detected.

After obtaining the corresponding target slice data by slicing the target three-dimensional limb model and detecting and calculating the volume parameter corresponding to each piece of target slice data, the volume parameters of each piece of target slice data are integrated to obtain the overall volume parameter of the target three-dimensional limb model, i.e., the overall volume information of the limb to be detected. Subsequently, pieces of overall volume information of the limb to be detected, which are detected at different times, may be compared and analyzed to determine whether the corresponding limb to be detected has a limb volume change such as edema.

In the above medical detection device provided with at least four cameras, the cameras are arranged at preset intervals to form the ring, the lenses of the cameras face toward the central region of the ring, and the ring is configured for the limb to be detected to pass through. Upon the detection that the limb to be detected has passed through the interior of the ring surrounded by the cameras, all the cameras are controlled to simultaneously move along the limb to be detected and perform scanning. The deduplication and fusion processing as well as the three-dimensional modeling processing are performed based on the point cloud data obtained by scanning to obtain the target three-dimensional limb model. The slicing processing and the centroid analysis processing are performed based on the target three-dimensional limb model to determine the joint centroid point. The re-slicing processing is performed on the target three-dimensional limb model based on the joint centroid point to obtain the target slice data. The parameter calculation and the data integration are performed based on the target slice data to obtain the overall volume information of the limb to be detected. By adopting the above technical solutions, the overall volume information of the limb to be detected can be obtained simply by scanning with the medical detection device, thereby avoiding the technical problems of low working efficiency and large error caused by manual measurement. Moreover, the overall volume information of the limb to be detected can be obtained based on camera scanning, thereby improving the working efficiency of the limb volume detection. Moreover, the preliminary slicing is first performed to determine the joint centroid point, and the re-slicing is performed based on the joint centroid point, with slicing being carried out separately using the joint centroid point as a boundary, thereby improving the accuracy of slices and improving the accuracy of the volume calculation.

On the basis of the above embodiments, FIG. 7 is a schematic view showing a structure of an apparatus for limb volume detection provided by an embodiment of the present application. Referring to FIG. 7, the apparatus for limb volume detection provided by this embodiment specifically includes: a scanning module 21, a point cloud fusion module 22, a modeling module 23, a joint determination module 24, a re-slicing module 25, a first calculation module 26, and a second calculation module 27.

The scanning module 21 is configured to, upon a detection that the limb to be detected has passed through the interior of the ring surrounded by the cameras, control all cameras to simultaneously move along the limb to be detected and perform scanning to obtain point cloud data acquired by scanning with the plurality of cameras.

The point cloud fusion module 22 is configured to perform deduplication and fusion processing on the point cloud data based on a preset spatial calibration relationship to obtain fused point cloud data of the cameras. The preset spatial calibration relationship is a relative positional relationship between every two cameras.

The modeling module 23 is configured to perform three-dimensional modeling processing based on the fused point cloud data to obtain a target three-dimensional limb model.

The joint determination module 24 is configured to perform slicing processing and centroid analysis processing based on the target three-dimensional limb model to determine a joint centroid point.

The re-slicing module 25 is configured to perform re-slicing processing on the target three-dimensional limb model based on the joint centroid point to obtain target slice data.

The first calculation module 26 is configured to perform parameter calculation processing based on the target slice data, to obtain volume information of each piece of target slice data. The volume information includes a slice thickness and a cross-sectional area.

The second calculation module 27 is configured to perform integration processing on the volume parameters of each piece of target slice data to obtain overall volume information of the limb to be detected.

In an embodiment, the modeling module 23 includes a voxel grid construction sub-module, a voxelization processing sub-module, and a voxel mapping sub-module.

The voxel grid construction sub-module is configured to create an initial voxel grid based on a resolution of the fused point cloud data.

The voxelization processing sub-module is configured to perform voxelization processing on the fused point cloud data based on the initial voxel grid, so as to obtain first voxel data. The first voxel data includes vertices, voxel indices, and a voxel value corresponding to each voxel.

The voxel mapping sub-module is configured to map the first voxel data into a point cloud mesh, and perform color rendering processing based on a mapping result and a projection superposition result to generate the target three-dimensional limb model.

In an embodiment, the joint determination module 24 includes a first slicing sub-module, a first centroid calculation sub-module, a first reference line determination sub-module, and a joint determination sub-module.

The first slicing sub-module is configured to perform slicing processing on the target three-dimensional limb model along a preset direction at preset distances to obtain reference slice data.

The first centroid calculation sub-module is configured to calculate and determine a centroid point of each piece of reference slice data.

The first reference line determination sub-module is configured to connect the centroid point of the first piece of reference slice data with the centroid point of the last piece of reference slice data to obtain a first reference line.

The joint determination sub-module is configured to determine, among the centroid points of all the reference slice data, the centroid point farthest from the first reference line as the joint centroid point.

In an embodiment, the re-slicing module 25 includes a second reference line determination sub-module, a first re-slicing sub-module, a third reference line determination sub-module, a second re-slicing sub-module, and a stitching sub-module.

The second reference line determination sub-module is configured to connect the centroid point of the first piece of the reference slice data with the joint centroid point to obtain a second reference line.

The first re-slicing sub-module is configured to perform re-slicing processing on a first portion of the target three-dimensional limb model along the second reference line at preset intervals to obtain first target slice data. The first portion is a portion between starting point cloud of the first piece of the reference slice data and ending point cloud in the reference slice data corresponding to the joint centroid point.

The third reference line determination sub-module is configured to connect the joint centroid point with the centroid point of the last piece of reference slice data to obtain a third reference line.

The second re-slicing sub-module is configured to perform re-slicing processing on a second portion of the target three-dimensional limb model along the third reference line at preset intervals to obtain second target slice data. The second portion is a portion between starting point cloud in the reference slice data corresponding to the joint centroid point and ending point cloud of the last piece of reference slice data.

The stitching sub-module is configured to perform fusion and stitching processing on the first target slice data and the second target slice data using the joint centroid point as a stitching point to obtain the overall target slice data of the target three-dimensional limb model.

In an embodiment, the apparatus for limb volume detection further includes a slicing module, a first centroid calculation module, a second centroid calculation module, a centroid point mapping module, a distance calculation module, and a target model determination module.

The modeling module 23 is further configured to perform two scans and two three-dimensional modeling processing operations on the limb to be detected to obtain a first three-dimensional limb model and a second three-dimensional limb model, respectively.

The slicing module is configured to perform slicing processing on the first three-dimensional limb model and the second three-dimensional limb model respectively along a preset direction at preset intervals, so as to obtain first slice data corresponding to the first three-dimensional limb model and second slice data corresponding to the second three-dimensional limb model.

The first centroid calculation module is configured to perform centroid point calculation processing on the first slice data to obtain first centroid point data. The first centroid point data includes the centroid points of all the first slice data.

The second centroid calculation module is configured to perform centroid point calculation processing on the second slice data to obtain second centroid point data. The second centroid point data includes the centroid points of all the second slice data.

The centroid point mapping module is configured to map the first centroid point data and the second centroid point data into a same plane for curve plotting to obtain a first curve and a second curve, respectively.

The distance calculation module is configured to perform distance calculation processing based on the first curve and the second curve to obtain a Euclidean distance between the first curve and the second curve.

The target model determination module is configured to, when the Euclidean distance does not exceed a preset value, determine the first three-dimensional limb model as the target three-dimensional limb model.

The target model determination module is further configured to, when the Euclidean distance exceeds the preset value, perform re-scanning and three-dimensional re-modeling processing on the limb to be detected until the Euclidean distance does not exceed the preset value, and determine the corresponding first three-dimensional limb model as the target three-dimensional limb model.

In an embodiment, the apparatus for limb volume detection further includes a calibration scanning module 21, a first position calibration module, a second position calibration module, a third position calibration module, and a spatial position calibration module.

The calibration scanning module 21 is configured to, upon a detection that the limb to be detected has passed through the interior of the ring surrounded by the cameras, control all the cameras to simultaneously move along the calibration object and perform scanning, so as to obtain reference point cloud data.

The first positional relationship calibration module is configured to compare the reference point cloud data with standard point cloud data to determine a first positional relationship of each camera relative to the calibration object. The standard point cloud data is obtained by performing point cloud calibration and correction on the calibration object.

The second position calibration module is configured to perform grouped calibration on the spatial positional relationships of the cameras based on the first positional relationship, so as to determine a second positional relationship between the cameras within the corresponding group.

The third position calibration module is configured to perform inter-group registration processing on the spatial positional relationships of the cameras based on the first positional relationship and the second positional relationship, so as to obtain a third relative positional relationship between every two groups.

The spatial position calibration module is configured to perform pairwise calibration processing on the spatial positional relationships of the cameras based on the first positional relationships, the second positional relationships, and the third relative positional relationship, so as to obtain the preset spatial calibration relationships between the cameras.

The apparatus for limb volume detection provided by the embodiments of the present application can be used to perform the method for limb volume detection provided by the above embodiments, and has corresponding functions and beneficial effects.

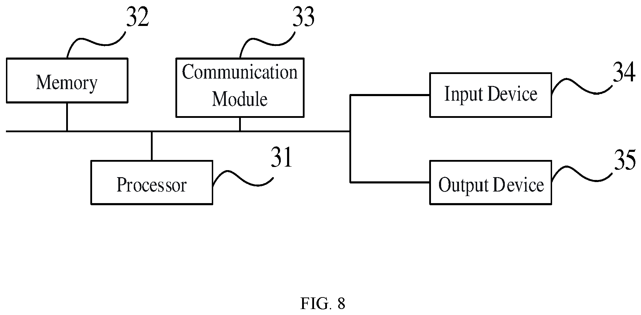

An embodiment of the present application provides a device for limb volume detection. Referring to FIG. 8, the device for limb volume detection includes: a processor 31, a memory 32, a communication module 33, an input device 34, and an output device 35. The number of processors in the device for limb volume detection may be one or more, and the number of memories in the device for limb volume detection may be one or more. The processor, the memory, the communication module, the input device, and the output apparatus of the device for limb volume detection may be connected via a bus or in another manner.

The memory 32, as a computer-readable storage medium, may be used to store software programs, computer-executable programs, and modules, such as the program instructions/modules corresponding to the method for limb volume detection described in any embodiment of the present application (for example, the scanning module, the point cloud fusion module, the modeling module, the joint determination module, the re-slicing module, the first calculation module, and the second calculation module in the apparatus for limb volume detection). The memory may mainly include a program storage area and a data storage area. The program storage area may store an operating system and an application program required by at least one function. The data storage area may store data created depending on the use of the device, and the like. In addition, the memory may include a high-speed random-access memory, and may further include a non-volatile memory, such as at least one magnetic disk storage device, a flash memory device, or any other non-volatile solid-state storage device. In some examples, the memory may further include a memory remotely arranged relative to the processor, and these remote memories may be connected to the device via a network. Examples of the above network include, but are not limited to, the Internet, an intranet, a local area network, a mobile communication network, and combinations thereof.

The communication module 33 is configured for data transmission.

The processor 31 executes various functional applications and data processing of the device by running the software programs, instructions, and modules stored in the memory, i.e., implements the method for limb volume detection described above.

The input device 34 may be configured to receive input numerical or character information and generate key signal inputs related to user settings and function control of the device. The output device 35 may include a display device such as a display screen.

The device for limb volume detection provided above can be used to perform the method for limb volume detection provided by the above embodiments, and has corresponding functions and beneficial effects.

An embodiment of the present application further provides a storage medium having computer-executable instructions stored thereon. The computer-executable instructions, when executed by a computer processor, are used to perform a method for limb volume detection. The method for limb volume detection includes: upon a detection that the limb to be detected has passed through the interior of the ring surrounded by the cameras, controlling all cameras to simultaneously move along the limb to be detected and perform scanning to obtain point cloud data acquired by scanning with the plurality of cameras; performing deduplication and fusion processing on the point cloud data based on a preset spatial calibration relationship to obtain fused point cloud data of the cameras, wherein the preset spatial calibration relationship is a relative positional relationship between every two cameras; performing three-dimensional modeling based on the fused point cloud data to obtain a target three-dimensional limb model; performing slicing processing and centroid analysis processing based on the target three-dimensional limb model to determine a joint centroid point; performing re-slicing processing on the target three-dimensional limb model based on the joint centroid point to obtain target slice data; performing parameter calculation processing based on the target slice data to obtain volume information of each piece of target slice data, wherein the volume information includes a slice thickness and a cross-sectional area; and performing integration processing on the volume parameters of each piece of target slice data to obtain overall volume information of the limb to be detected.

Storage medium - any type of memory device or storage device. The term "storage medium" is intended to include: installation media, such as a CD-ROM, a floppy disk, or a tape device; computer system memory or random access memory, such as DRAM, DDR RAM, SRAM, EDO RAM, Rambus RAM, etc.; non-volatile memory, such as flash memory, magnetic media (e.g., a hard disk or optical storage); registers or other similar types of memory elements, etc. The storage medium may also include other types of memory or combinations thereof. In addition, the storage medium may be located in a first computer system in which the program is executed, or may be located in a different second computer system that is connected to the first computer system via a network (such as the Internet). The second computer system may provide program instructions to the first computer for execution. The term "storage medium" may include two or more storage media residing in different locations (for example, in different computer systems connected via a network). The storage medium may store program instructions (for example, embodied as a computer program) executable by one or more processors.

Of course, in the storage medium storing computer-executable instructions provided by the embodiments of the present application, the computer-executable instructions are not limited to the method for limb volume detection as described above, and may also perform related operations in the method for limb volume detection provided by any embodiment of the present application.

The apparatus for limb volume detection, the storage medium, and the device for limb volume detection provided in the above embodiments can perform the method for limb volume detection provided by any embodiment of the present application. For technical details not described in detail in the above embodiments, reference may be made to the method for limb volume detection provided by any embodiment of the present application.

Described above are the preferred embodiments of the present application and the applied technical principles. The present application is not limited to the specific embodiments described herein, and for those skilled in the art, various obvious changes, readjustments, and substitutions can be made without departing from the scope of protection of the present application. Therefore, although the present application has been described in details through the above embodiments, the present application is not limited to the above embodiments, and may further include more other equivalent embodiments without departing from the concept of the present application, while the scope of the present application is determined by the scope of the claims.

## Claims

1. A method for limb volume detection, for use in a medical detection device, wherein the medical detection device is provided with at least four cameras arranged circumferentially at preset intervals around a ring, lenses of the cameras face toward a central region of the ring, and the ring is configured for a limb to be detected to pass through; and the method comprises:
upon a detection that the limb to be detected has passed through the interior of the ring surrounded by the cameras, controlling all cameras to simultaneously move along the limb to be detected and perform scanning to obtain point cloud data acquired by scanning with a plurality of cameras;
performing deduplication and fusion processing on the point cloud data based on a preset spatial calibration relationship to obtain fused point cloud data of the cameras, and the preset spatial calibration relationship being a relative positional relationship between every two cameras;
performing three-dimensional modeling processing based on the fused point cloud data to obtain a target three-dimensional limb model;
performing slicing processing and centroid analysis processing based on the target three-dimensional limb model to determine a joint centroid point;
performing re-slicing processing on the target three-dimensional limb model based on the joint centroid point to obtain target slice data;
performing parameter calculation processing based on the target slice data to obtain volume information of each piece of target slice data, and the volume information comprising a slice thickness and a cross-sectional area; and
performing integration processing on volume parameters of each piece of target slice data to obtain overall volume information of the limb to be detected.

2. The method according to claim 1, wherein performing the deduplication and fusion processing on the point cloud data based on the preset spatial calibration relationship to obtain the fused point cloud data of all the cameras, and the preset spatial calibration relationship being the relative positional relationship between every two cameras, comprises:
determining an overlapping region in regions corresponding to the point cloud data based on the preset spatial calibration relationship; and
performing the deduplication processing on the point cloud data of the overlapping region to obtain the fused point cloud data, wherein the fused point cloud data comprises point cloud data of a non-overlapping region, and the processed point cloud data of the overlapping region obtained after the deduplication processing.

3. The method according to claim 1, wherein performing the three-dimensional modeling processing based on the fused point cloud data to obtain the target three-dimensional limb model comprises:
creating an initial voxel grid based on a resolution of the fused point cloud data;
performing voxelization processing on the fused point cloud data based on the initial voxel grid to obtain first voxel data, and the first voxel data comprising vertices, voxel indices, and a voxel value corresponding to each voxel; and
mapping the first voxel data into a point cloud mesh, and performing color rendering processing based on a mapping result and a projection superposition result to generate the target three-dimensional limb model.

4. The method according to claim 1, wherein performing the slicing processing and the centroid analysis processing based on the target three-dimensional limb model to determine the joint centroid point comprises:
performing the slicing processing on the target three-dimensional limb model along a preset direction at preset distances to obtain reference slice data;
calculating and determining a centroid point of each piece of the reference slice data;
connecting a centroid point of a first piece of the reference slice data with a centroid point of a last piece of the reference slice data to obtain a first reference line; and
determining, among centroid points of all the reference slice data, a centroid point farthest from the first reference line as the joint centroid point.

5. The method according to claim 4, wherein performing the re-slicing processing on the target three-dimensional limb model based on the joint centroid point to obtain the target slice data comprises:
connecting the centroid point of the first piece of the reference slice data with the joint centroid point to obtain a second reference line;
performing the re-slicing processing on a first portion of the target three-dimensional limb model along the second reference line at preset intervals to obtain first target slice data, wherein the first portion is a portion between starting point cloud of the first piece of the reference slice data and ending point cloud in the reference slice data corresponding to the joint centroid point;
connecting the joint centroid point with the centroid point of the last piece of the reference slice data to obtain a third reference line;
performing the re-slicing processing on a second portion of the target three-dimensional limb model along the third reference line at preset intervals to obtain second target slice data, wherein the second portion is a portion between starting point cloud in the reference slice data corresponding to the joint centroid point and ending point cloud of the last piece of the reference slice data; and
performing fusion and stitching processing on the first target slice data and the second target slice data using the joint centroid point as a stitching point to obtain overall target slice data of the target three-dimensional limb model.

6. The method according to claim 1, wherein before performing the slicing processing and the centroid analysis processing based on the target three-dimensional limb model to determine the joint centroid point, the method comprises:
performing two scans and two three-dimensional modeling processing operations on the limb to be detected to obtain a corresponding first three-dimensional limb model and a second three-dimensional limb model, respectively;
performing the slicing processing on the first three-dimensional limb model and the second three-dimensional limb model respectively along a preset direction at preset intervals, to obtain first slice data corresponding to the first three-dimensional limb model and second slice data corresponding to the second three-dimensional limb model;
performing centroid point calculation processing on the first slice data to obtain first centroid point data, wherein the first centroid point data comprises centroid points of all the first slice data;
performing centroid point calculation processing on the second slice data to obtain second centroid point data, wherein the second centroid point data comprises centroid points of all the second slice data;
mapping the first centroid point data and the second centroid point data into a same plane for curve plotting to obtain a first curve and a second curve, respectively;
performing distance calculation processing based on the first curve and the second curve to obtain a Euclidean distance between the first curve and the second curve;
determining the first three-dimensional limb model as the target three-dimensional limb model when the Euclidean distance does not exceed a preset value; and
performing re-scanning and three-dimensional re-modeling processing on the limb to be detected when the Euclidean distance exceeds the preset value, until the Euclidean distance does not exceed the preset value, and determining a corresponding first three-dimensional limb model as the target three-dimensional limb model.

7. The method according to claim 1, wherein the method further comprises:
upon a detection that a calibration object has passed through the interior of the ring surrounded by the cameras, controlling all the cameras to simultaneously move along the calibration object and perform scanning to obtain reference point cloud data;
comparing the reference point cloud data with standard point cloud data to determine a first positional relationship of each camera relative to the calibration object, wherein the standard point cloud data is obtained by performing point cloud calibration and correction on the calibration object;
performing grouped calibration on the spatial positional relationships of the cameras based on the first positional relationship to determine a second positional relationship between the cameras within a corresponding group;
performing inter-group registration processing on the spatial positional relationships of the cameras based on the first positional relationship and the second positional relationship to obtain a third relative positional relationship between every two groups; and
performing pairwise calibration processing on the spatial positional relationships of the cameras based on the first positional relationships, the second positional relationships, and the third relative positional relationship to obtain the preset spatial calibration relationships between all the cameras.

8. An apparatus for limb volume detection, for use in a medical detection device, wherein the medical detection device is provided with at least four cameras arranged circumferentially at preset intervals around a ring, lenses of the cameras face toward a central region of the ring, and the ring is configured for a limb to be detected to pass through; and the apparatus comprises:
a scanning module, configured to, upon a detection that the limb to be detected has passed through the interior of the ring surrounded by the cameras, control all cameras to simultaneously move along the limb to be detected and perform scanning to obtain point cloud data acquired by scanning with a plurality of cameras;
a point cloud fusion module, configured to perform deduplication and fusion processing on the point cloud data based on a preset spatial calibration relationship to obtain fused point cloud data of the cameras, wherein the preset spatial calibration relationship is a relative positional relationship between every two cameras;
a modeling module, configured to perform three-dimensional modeling processing based on the fused point cloud data to obtain a target three-dimensional limb model;
a joint determination module, configured to perform slicing processing and centroid analysis processing based on the target three-dimensional limb model to determine a joint centroid point;
a re-slicing module, configured to perform re-slicing processing on the target three-dimensional limb model based on the joint centroid point to obtain target slice data;
a first calculation module, configured to perform parameter calculation processing based on the target slice data to obtain volume information of each piece of target slice data, wherein the volume information comprises a slice thickness and a cross-sectional area; and
a second calculation module, configured to perform integration processing on volume parameters of each piece of target slice data to obtain overall volume information of the limb to be detected.

9. A device for limb volume detection, comprising: a memory and one or more processors;
wherein the memory is configured to store one or more programs;
the one or more programs, when executed by the one or more processors, cause the one or more processors to perform the method according to any one of claims 1 to 7.

10. A storage medium, having computer-executable instructions stored thereon, wherein the computer-executable instructions, when executed by a processor, are used to perform the method according to any one of claims 1 to 7.
